# EUROPEAN PATENT SPECIFICATION

(11) **EP 0 766 971 B1**
(45) Date of publication and mention of the grant of the patent: **03.04.2002**
(21) Application number: 96830480.8
(22) Date of filing: 25.09.1996
(51) Int. Cl.: A61L 2/26, A61L 2/18

(54) **A device for holding dental handpieces in the apparatus where they are disinfected and sterilized**
Vorrichtung zur Aufnahme von zahnärztlichen Handstücken in einem Desinfektions- und Sterilisiergerät
Dispositif de maintien de pièces à main dentaire dans un appareil de désinfection et stérilisation

(30) Priority: 28.09.1995 IT BO950141 U
(43) Date of publication of application: 09.04.1997
(73) Proprietor: CASTELLINI S.p.A., I-40013 Castel Maggiore (Bologna) (IT)
(72) Inventor: Castellini, Franco, 40123 Bologna (IT)
(74) Representative: Lanzoni, Luciano

(56) References cited:
- EP-A- 0 321 415
- EP-A- 0 345 228
- CH-A- 621 270
- FR-A- 2 498 933
- US-A- 4 050 894

## Description

The present invention relates to a dental unit with a device for holding dental handpieces in the apparatus where they are disinfected and sterilized.

In order to maintain a high standard of hygiene, it has become regular practice in dental surgeries to sterilize dental instruments and handpieces between one patient and the next or at the start or end of a day's work.

One method of sterilizing dental equipment, used preferably when the dental unit is not in operation, envisages an intermittent cycle (for example, see patent applications EP - 111.249 and EP -317.521, the latter being by the same Applicant as the present), during which the mains water supply to the handpieces is turned off and sterilizing liquid is fed into the handpiece pipes through a "dedicated" branch of the system equipped with an independent tank. After a defined time, which depends on the degree of sterilization required and on the amount of sterilizing liquid used, the water supply is turned on again and the polluted waste liquid is rinsed out through the handpieces themselves.

To recover the liquid and avoid dripping during the feeding in or rinsing out of the sterilizing liquid, the instruments, before the sterilization procedure is started, are placed in a specially designed container where the sterilizing liquid, polluted by the materials removed from the handpieces, collects.

Up to the present time, the containers used for this purpose (see also patent EP-345.228) are not made as part of the dental units but are designed to be attached to the dental units near the drainage section, usually at the top of a column of the dental unit where the cuspidor bowl and the rinsing tumbler support plate are located.

Containers of this kind consist of a tub closed at the top by a lid having in it a plurality of openings designed to accommodate the handpieces and instruments to be treated. The tub is usually equipped with appropriate drains placed at the drainage holes of the cuspidor bowl (see also patent DE-36.11.327) or which are connected to the main suction device of the dental unit.

The tub lid has tubular projections extending into the container, each of which is coupled with a cap that closes the projection and that has a handpiece retaining web and drainage holes, when the projection is occupied by a handpiece: this provides a firm holder for the handpieces to be treated, that occupies a limited space and having small holes (made in the caps) for draining off the waste liquid which thus flows to the bottom of the tank and out through the main drain.

In the structural design of a dental unit of the latest generation, the container is integral with the upper surface having the holes for the handpieces and is incorporated into the structure of the dental unit itself. However, while a solution of this kind has obviously made it much more convenient to run sterilization cycles, it has made inconvenient all the solutions based on capped tubular projections, since the inside of the container is no longer easily accessible.

For this purpose, the Applicant has created a dental unit with a device designed to hold handpieces in the sterilisation and disinfection apparatus, that in suitable for autoclaving and that can be quickly and easily fitted to and removed from the seats of the container forming part of the dental unit.

The technical characteristics of the disclosure are laid out in the claims below and its advantages are apparent from the detailed description which follows, with reference to the accompanying drawings, which illustrate a preferred embodiment of the invention by way of example and in which:
- Figure 1 shows the handpiece holding device disclosed, applicable to the sterilization and disinfection apparatus of dental units, in a perspective view which illustrates a part of the dental unit;
- Figures 2 and 3 are, respectively, a side view and a top plan view of the device shown in Fig. 1;
- Figure 4 is a partial cross sectional view of the device disclosed applied to the dental unit illustrated in Fig. 1;
- Figure 5 is a perspective exploded view, with some parts cut away in order to better illustrate others, of the part of the dental unit shown in Fig. 1.

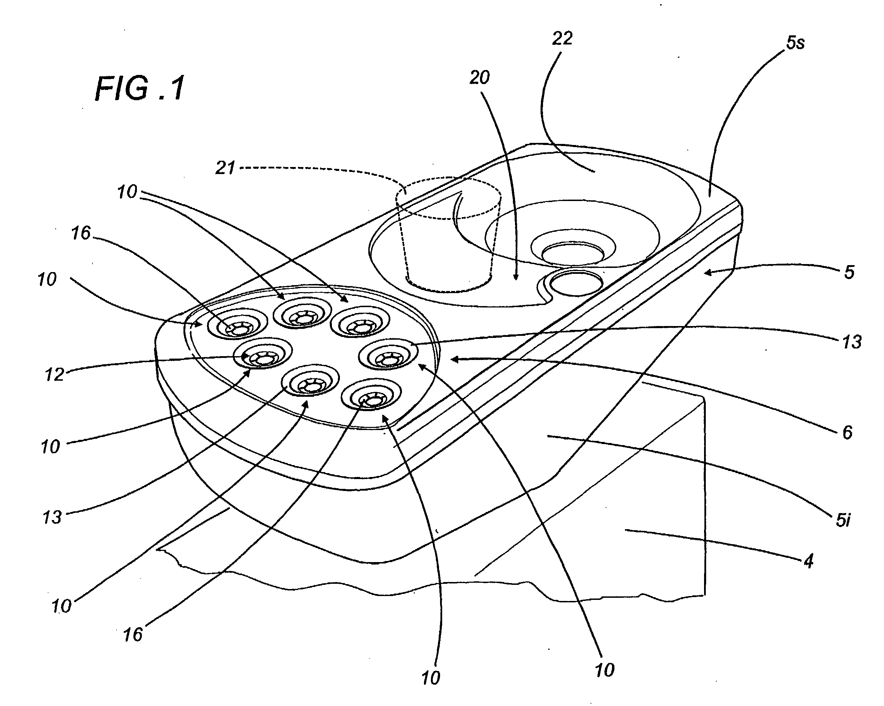

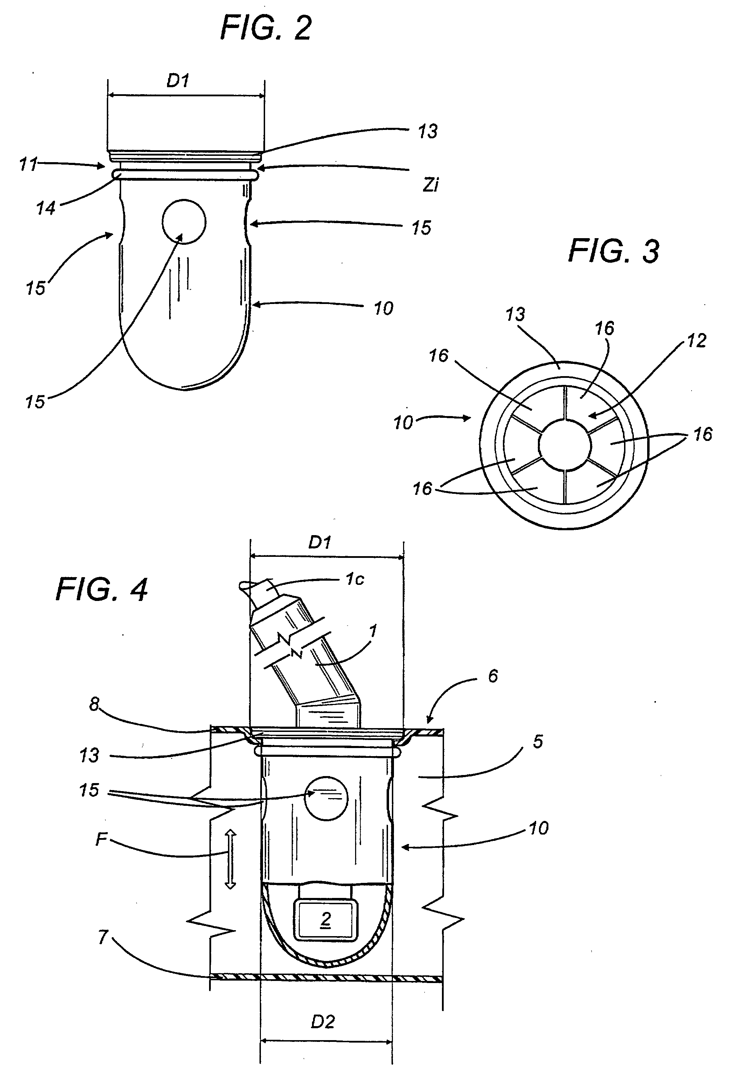

With reference to the accompanying drawings, in particular Figs. 1 and 4, the device disclosed is designed to hold dental instruments or handpieces 1 equipped with a work head 2 and is applied to the apparatus used for disinfecting and sterilizing the said handpieces.

Such apparatus forms part of a dental unit including a main base 4 with an upper portion 5 equipped with a container 6 consisting of a tub 7 closed by a lid 8 having a plurality of holes 9 each of which can accommodate a handpiece 1 at rest so as to allow the draining off of the polluted liquids flowing out through the feed hose 1c of the handpiece 1.

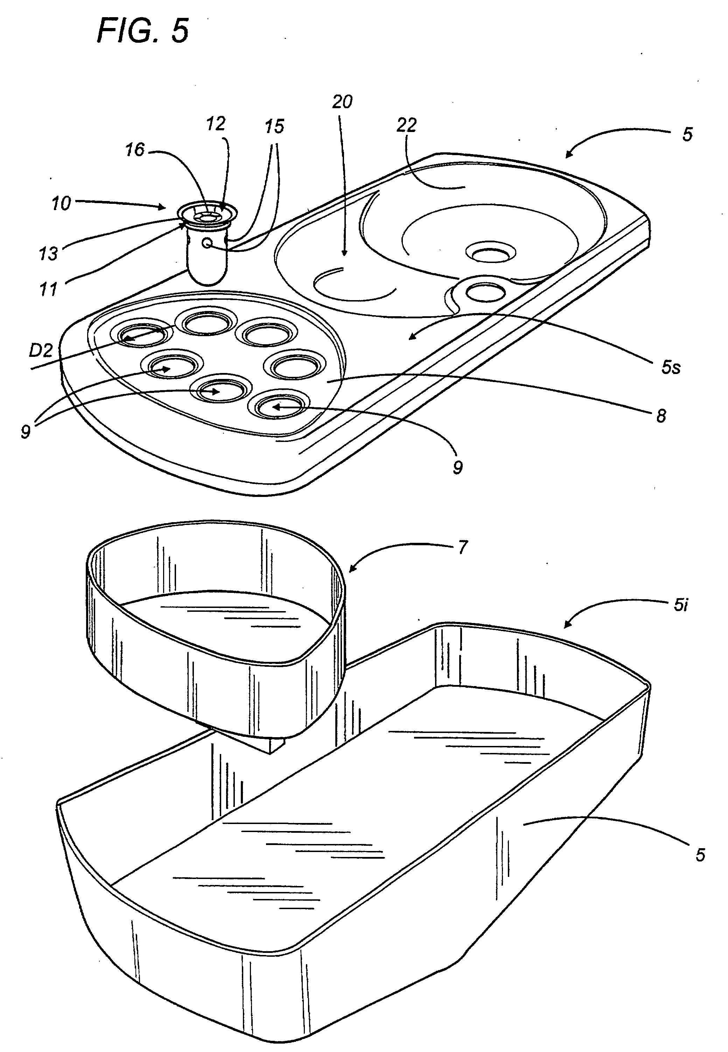

In the embodiment of the disclosure as illustrated in Figs. 1 and 5, the tub 7 and the lid 8 are integral with the dental unit's upper portion 5 which also accommodates a plate 20, for supporting a rinsing tumbler 21, and a cuspidor bowl 22: in particular, the tub 7 is located inside the lower section 5i of the portion 5, whilst the lid 8 forms an integral part of the upper section 5s of the portion itself, where the plate 20 and the cuspidor bowl 22 are made.

From the outside of the container 6, tubular elements 10, closed at one end to form caps, can be inserted into, and removed from, the said holes 9 (see arrow F, Fig. 4). Each tubular element 10 is made of a flexible material, suitable for autoclaving (for example, a heat-resistant elastomer) and is equipped with means 11 for positioning it and fastening it to the lid 8, the said means being located at the open end of the tubular element 10. As shown in Fig. 1, there may be as many tubular elements 10 as there are holes 9 in the lid 8.

In Figs. 1 and 4, the numeral 12 indicates means for retaining the handpiece 1, located inside the tubular element 10. The retaining means 12 consist of a plurality of flexible flaps 16 made at the top end of the tubular element 10; the flaps 16 extend inwards towards the centre of the tubular element 10, leaving an opening at the centre which, when the element is not being used, is smaller in diameter than the diameter of the handpiece 1 held by the tubular element 10: in this way, the handpiece is held in a slightly raised position so that its work head 2 does not touch the bottom of the tubular element 10.

The said positioning and fastening means 11 consist of a circular crown 13 made at the open end of the tubular element 10 and having a diameter D1 greater than the diameter D2 of the holes 9 in such a way as to form a surface to support the tubular element 10 applied to it. On the underside of the crown 13, there is a ring 14 extending round the circumference of the tubular element 10 and, together with the crown 13, defining an area, labelled Zi, that mates with the outer edge of the corresponding hole 9.

The tubular element 10 also has a plurality of through holes 15 made radially in its surface immediately below the said ring 14 in order to allow the polluted liquids flowing out of the handpiece 1 to be drained off.

The disclosure therefore provides a convenient holder for handpieces 1 that is easy to attach and remove from the container 6 which forms an integral part of the upper portion 5 of the dental unit. Hygiene is also guaranteed by the fact that the tubular elements 10 can be autoclaved whenever necessary, since they themselves come into contact with the polluted liquids flowing out of the handpiece and into the drain at the bottom of the container 6. Moreover, all this is possible without altering the ergonomic and structural configuration of the container 6.

## Claims

1. Dental unit with a device for holding dental instruments or handpieces (1) equipped with a work head (2) which are to be disinfected and sterilized said dental unit comprising a main base (4) with an upper portion (5) equipped with a container (6) consisting of a tub (7) closed by a lid (8) having a plurality of holes (9) each of which can accommodate a handpiece (1) at rest so as to allow the draining off of the polluted liquids flowing out through the feed hose (1c) of the handpiece (1); the device comprising tubular elements (10), housed in the container and closed at one end, designed to hold at least the head (2) of the handpieces (1), the said dental unit being **characterized in** chat the tub (7) and the lid (8) are integral with the upper portion (5) and **in that** tubular elements (10) can be inserted into the holes (9) from the outside of the container (6), each tubular element (10) being made of a flexible material, suitable for autoclaving and being equipped, at its open end, with means (11) for positioning and fastening it to the lid (8).

2. The dental unit according to claim 1 **characterized in that** the said tubular element (10) can be inserted from the outside of the said container (6) into each of the said holes (9) and constitutes a cap equipped at its free end with means (12) for retaining the handpiece (1) inside the cap itself.

3. The dental unit according to claim 1 **characterized in that** the said positioning and fastening means (11) consist of a circular crown (13) made at the open end of the tubular element (10) and having a diameter (D1) greater than the diameter (D2) of the holes (9); there being, on the underside of the crown 13, a ring (14) extending round the circumference of the said tubular element (10) and, together with the crown (13), defining an area (Zi) that mates with the outer edge of the corresponding hole (9).

4. The dental unit according to claim 1 **characterized in that** the said tubular element (10) has a plurality of through holes (15) made radially in its surface below the said positioning and fastening means (11) and designed to allow the said polluted liquids to be drained off.

5. The dental unit according to claim 3 **characterized in that** the said retaining means (12) consist of a plurality of flexible flaps (16) made at the top end of the tubular element; the flaps (16) extending inwards towards the centre of the tubular element (10) and leaving an opening at the centre which, when the element is not being used, is smaller in diameter than the diameter of the handpiece (1) held by the tubular element (10) itself.

## Patentansprüche

1. Dentaleinheit mit einer Vorrichtung zum Halten von zahnärztlichen Instrumenten oder Handstücken (1), die mit einem Arbeitskopf (2) versehen sind und desinfiziert und sterilisiert werden sollen, wobei die genannte Dentaleinheit einen Hauptsockel (4) mit einem oberen Abschnitt (5) enthält, ausgestattet mit einem Behälter (6), der aus einer Wanne (7) besteht, verschlossen durch einen Deckel (8) mit einer Anzahl von Öffnungen (9), von denen jede ein Handstück (1) in seinem Ruhezustand aufnehmen kann, um das Abfliessen der verschmutzten Flüssigkeiten zu erlauben, die aus dem Zuführschlauch (1c) des Handstückes (1) ausfliessen; wobei die Vorrichtung rohrförmige Elemente (10) enthält, die in dem Behälter aufgenommen werden und, an einem Ende verschlossen, dazu dienen, wenigstens einen Kopf (2) der Handstücke (1) zu halten, wobei die genannte Dentaleinheit **dadurch gekennzeichnet ist, dass** die Wanne (7) und der Deckel (8) in einem Stück mit dem oberen Abschnitt (5) gearbeitet sind, und dadurch, dass die rohrförmigen Elemente (10) von aussen her in den Behälter (6) eingesetzt werden können, und wobei jedes rohrförmige Element (10) aus einem für die Autoklave geeigneten flexiblen Material hergestellt und an seinem oberen Ende mit Mitteln (11) versehen ist, um es an dem Deckel (8) zu positionieren und zu befestigen.

2. Dentaleinheit nach Patentanspruch 1, **dadurch gekennzeichnet, dass** das genannte rohrförmige Element (10) von aussen her in den genannten Behälter (6) eingesetzt werden kann, und zwar in jede der genannten Öffnungen (9), und eine Kappe bildet, die an ihrem freien Ende mit Mitteln (12) zum Halten des Handstückes (1) im Inneren der Kappe selbst versehen ist.

3. Dentaleinheit nach Patentanspruch 1, **dadurch gekennzeichnet, dass** die genannten Positionier- und Befestigungsmittel (11) aus einer kreisförmigen Krone (13) bestehen, die an dem offenen Ende des rohrförmigen Elementes (10) angearbeitet ist und einen Durchmesser (D1) aufweist, der grösser ist als der Durchmesser (D2) der Öffnungen (9); wobei an der Unterseite der Krone (13) ein Ring (14) vorgesehen ist, der sich entlang dem Umlauf des genannten rohrförmigen Elementes (10) erstreckt und zusammen mit der Krone (13) einen Bereich (Zi) bildet, der mit dem äusseren Rand den entsprechenden Öffnung (9) ineinander greift.

4. Dentaleinheit nach Patentanspruch 1, **dadurch gekennzeichnet, dass** das genannte rohrförmige Element (10) eine Anzahl von durchgehenden Bohrungen (15) aufweist, eingearbeitet radial in seine Oberfläche unterhalb der genannten Positionier- und Befestigungsmittel (11) und dazu bestimmt, das Abfliessen der genannten verschmutzten Flüssigkeit zu erlauben.

5. Dentaleinheit nach Patentanspruch 3, **dadurch gekennzeichnet, dass** die genannten Haltemittel (12) aus einer Anzahl von flexiblen Flügeln (16) bestehen, die sich an dem oberen Ende des rohrförmigen Elementes befinden; wobei sich die Flügel (16) nach innen und zur Mitte des rohrförmigen Elementes (10) hin erstrecken und in der Mitte eine Öffnung frei lassen, welche, wenn das Element nicht benutzt wird, im Durchmesser kleiner ist als der Durchmesser des Handstückes (1), das von dem rohrförmigen Element (10) selbst getragen wird.

## Revendications

1. Un bloc dentaire avec un dispositif de maintien d'instruments ou pièces à main dentaires (1) pourvues d'une tête de travail (2) et devant être désinfectées et stérilisées, ledit bloc dentaire comprenant une base principale (4) avec une portion supérieure (5) équipée d'un récipient (6) consistant en un bac (7) fermé par un couvercle (8) ayant une pluralité de trous (9) pouvant chacun recevoir une pièce à main (1) au repos, de manière à permettre l'évacuation des liquides pollués s'écoulant à travers le flexible d'alimentation (1c) de la pièce à main (1) ; le dispositif comprenant des éléments tubulaires (10), logés dans le récipient et fermés à une extrémité, destinés à maintenir au moins la tête (2) des pièces à main (1), ledit bloc dentaire étant **caractérisé en ce que** le bac (7) et le couvercle (8) font partie intégrante de la portion supérieure (5) et **en ce que** les éléments tubulaires (10) peuvent être introduits dans les trous (9) de l'extérieur du récipient (6), chaque élément tubulaire (10) étant réalisé dans un matériau flexible, pouvant passer à l'autoclave, et étant équipé, au niveau de son extrémité ouverte, de moyens (11) destinés à le positionner et à le fixer au couvercle (8).

2. Le bloc dentaire selon la revendication 1, **caractérisé en ce que** ledit élément tubulaire (10) peut être introduit de l'extérieur du récipient (6) dans chacun des trous (9) susmentionnés et constitue un bouchon pourvu, au niveau de son extrémité libre, de moyens (12) destinés à retenir la pièce à main (1) à l'intérieur du bouchon lui-même.

3. Le bloc dentaire selon la revendication 1, **caractérisé en ce que** lesdits moyens (11) de positionnement et de fixation consistent en une couronne circulaire (13) réalisée au niveau de l'extrémité ouverte de l'élément tubulaire (10) et ayant un diamètre (D1) supérieur au diamètre (D2) des trous (9) ; une bague (14) étant prévue inférieurement à la couronne (13), autour de la circonférence de l'élément tubulaire (10), et définissant, avec cette même couronne (13), une zone (Zi) qui s'emboîte avec le bord extérieur du trou (9) correspondant.

4. Le bloc dentaire selon la revendication 1, **caractérisé en ce que** ledit élément tubulaire (10) a une pluralité de trous débouchants (15) réalisés radialement dans sa surface en dessous des moyens de positionnement et de fixation (11) susmentionnés et destinés à permettre aux liquides pollués d'être évacués.

5. Le bloc dentaire selon la revendication 3, **caractérisé en ce que** lesdits moyens de retenue (12) consistent en une pluralité de lamelles flexibles (16) réalisées au niveau de l'extrémité supérieure de l'élément tubulaire ; les lamelles (16) s'étendant vers l'intérieur vers le centre de l'élément tubulaire (10) et laissant une ouverture au centre qui, lorsque l'élément n'est pas utilisé, a un diamètre inférieur au diamètre de la pièce à main (1) maintenue par l'élément tubulaire (10) lui-même.
